# EUROPEAN PATENT APPLICATION

(11) **EP 1 336 408 A1**
(43) Date of publication of application: **20.08.2003**
(21) Application number: 01997305.6
(22) Date of filing: 21.11.2001
(51) Int. Cl.: A61K 31/426, A61K 9/08, A61K 47/16, C07D 277/54, A61P 1/04

(54) **WATER-SOLUBLE LIQUID INTERNAL MEDICINE**

(30) Priority: 24.11.2000 JP 2000357701
(71) Applicant: YAMANOUCHI PHARMACEUTICAL CO. LTD., Tokyo 103-8411 (JP)
(72) Inventor: HASHIMOTO, Yoshimi, 17-1, Hasune 3-chome, Itabashi-ku, Tokyo (JP); FURUYA, Nobuyoshi, 180, Ozumi, Yaizu-shi, Shizuoka 425-0072 (JP); KOJIMA, Haruyoshi, 180, Ozumi, Yaizu-shi, Shizuoka 425-0072 (JP)
(74) Representative: Bates, Philip Ian
(86) International application number: JP0110175
(87) International publication number: WO02041892

(57) **Abstract**

A water-soluble oral solution comprising famotidine and edetic acid or a salt thereof.

## Description

### TECHNICAL FIELD

The present invention relates to a water-soluble oral solution of famotidine.

### BACKGROUND ART

Tablets and powders containing famotidine as an active ingredient go on the market worldwide and widely used as a superior therapeutic agent for ulcer or gastritis, but its oral solutions have not gone on the market yet.

This is because famotidine is a basic compound, and in an acidic region, famotidine is soluble, but its stability is low, whereas in a neutral region where famotidine is stable, its solubility is extremely lowered. In addition, in oral solutions, a taste is an important issue different from injectable solutions. When the taste properties are taken into consideration, it is impossible to render the oral solutions weakly basic or basic.

JP-B-63-65047 (or Canadian Patent No. 1,184,495) describes that an aqueous solution for injection of 1 mg/mL of famotidine (pH: 5.3) as obtained by adding L-aspartic acid exhibits superior stability, as compared with those as obtained by adding maleic acid, fumaric acid, tartaric acid, citric acid, phthalic acid, or the like.

Further, U.S. Patent No. 5,650,421 describes that injections containing from 0.1 to 0.8 mg/mL of famotidine or its salt, whose pH is adjusted at from 5.7 to 6.4 by the addition of L-aspartic acid or the like, exhibited stability such that a residual rate after allowing to stand at room temperature for one year was 95% or more.

On the other hand, JP-A-11-193233 discloses injectable solutions containing a high concentration (from about 1 mg/mL to about 40 mg/mL) of famotidine and having a pH of from about 5.5 to about 7.5 as a stable injectable solution of famotidine, which are prepared by solubilizing famotidine with a water-soluble nonaqueous solvent such as polyethylene glycol, propylene glycol, ethanol, and glycerin and making the water-soluble nonaqueous solvent co-present with an acidic substance such as lactic acid, L-aspartic acid, L-glutamic acid, benzoic acid, citric acid, phosphoric acid, ascorbic acid, gluconic acid, acetic acid, and nicotinic acid, and describes that it is possible to stabilize famotidine in the solution at room temperature for a long period of time.

However, in applying the stabilization technologies as described in these patent documents to oral solutions as they are, there were involved problems as described below in detail.

That is, in order that the oral solutions are satisfied with the examination guidelines for approval of medicaments and endure the market circulation, their residual rate after preservation at room temperature for one year must be about 97%, a value of which is reduced into a residual rate after preservation at 40°C for 2 months.

The stability attained by the addition of L-aspartic acid as described in JP-B-63-65047 and U.S. Patent No. 5,650,421 is not satisfactory as shown in Test Example 1 described later.

In the working examples of JP-A-11-193233, in the injectable solution having a concentration of famotidine of 1 mg/mL, the residual rate after preservation at 40°C for 2 months is lowered to 96.6%. However, in oral solutions of bitter famotidine, taking into consideration the taste, it is desired that its concentration be about 1 mg/mL. Accordingly, the method as described in this patent document is not suitable. Further, JP-A-11-193233 describes that it is preferred to treat products such that they do not come into contact with oxygen, by, for example, blowing a nitrogen gas into a preparation or filling a space of ampoule with a nitrogen gas, and in all of the working examples of this patent document, nitrogen displacement is carried out. The nitrogen displacement requires its own step during the production steps, resulting in an increase of the production cost. Thus, such is not desired.

It is necessary to create a water-soluble oral solution of famotidine, which can be produced by inexpensive and simple production steps and has stability at room temperature for a long period of time such that it can endure the market circulation.

### DISCLOSURE OF THE INVENTION

The present inventor made extensive and intensive investigations with respect to the stabilization method. As a result, it has been unexpectedly found that an oral solution of a low concentration of famotidine, which is extremely stable so that a residual rate of famotidine after preservation at room temperature for 12 months is 97% or more, is obtained by merely adding edetic acid or a salt thereof, leading to accomplishment of the invention.

Specifically, the invention is concerned with a water-soluble oral solution comprising famotidine and edetic acid or a salt thereof. Preferably, the invention is concerned with a water-soluble oral solution comprising famotidine and edetic acid or a salt thereof and having a pH of from 5.5 to 7.0. More preferably, the invention is concerned with a water-soluble oral solution comprising famotidine and edetic acid or a salt thereof and having a pH of from 5.5 to 7.0 and a concentration of famotidine of from 0.025 to 4 mg/mL (most preferably from 0.2 to 2 mg/mL).

In the invention, a stable water-soluble oral solution is obtained by merely adding edetic acid or a salt thereof to adjust the pH without adding various additives, and nitrogen displacement is not particularly required, thereby enabling to make the production steps simple. Further, even in low-concentration solutions having a concentration of famotidine of 1 mg/mL or less, it is possible to ensure sufficient stability at room temperature such that the solutions endure the market circulation, i.e., the residual rate after preservation at room temperature for one year is 97% or more.

Famotidine form metabolites caused by hydrolysis of an aminosulfonylamidino group that is characteristically present in the chemical structure thereof into an aminocarbonyl group or an aminosulfonylaminocarbonyl group. Further, there is known a metabolite caused by oxidation of a sulfinyl group present in the chemical structure to form sulfoxide. In the invention, edetic acid or its salt not only functions as an antioxidant to suppress the formation of a metabolite caused by oxidation of famotidine but also suppresses the formation of metabolites caused by hydrolysis of famotidine, thereby achieving the stability of famotidine at a high level in the preservation at room temperature.

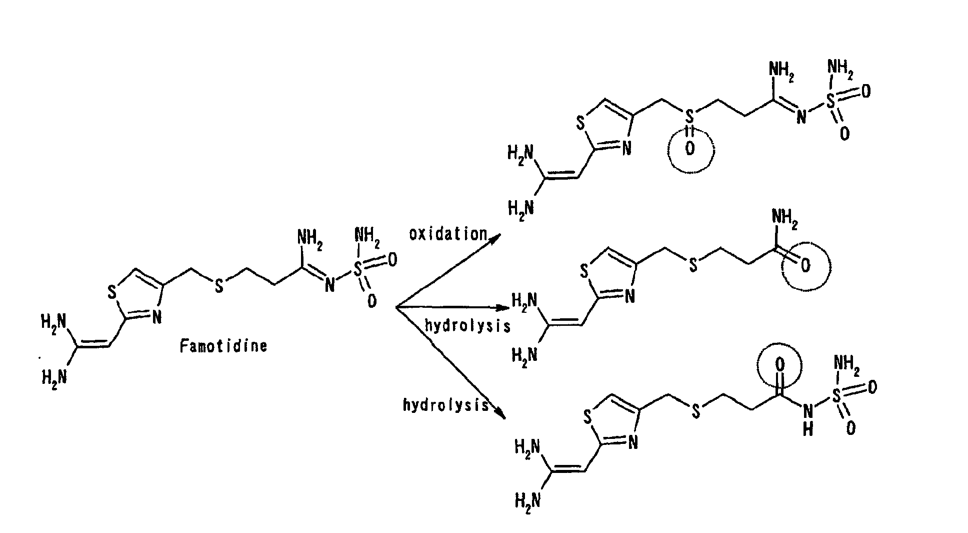

The invention will be described below in more detail.

In the water-soluble oral solution of the invention, the concentration of famotidine is preferably from 0.025 mg/mL to 4 mg/mL, and more preferably from 0.2 mg/mL to 2 mg/mL. This is because the usual dose of famotidine is from 5 mg to 20 mg, and the usual dose as an oral solution is from 2.5 mL to 200 mL.

As a raw material of the production of the water-soluble oral solution of the invention, an acid-addition salt of famotidine may be used in place of famotidine. Examples of the acid-addition salt of famotidine include salts with hydrochloric acid, hydrobromic acid, hydroiodic acid, sulfuric acid, fumaric acid, maleic acid, succinic acid, tartaric acid, and picric acid.

Edetic acid is also called ethylenediaminetetraacetic acid as another name.

In the invention, examples of the salt of edetic acid include a disodium salt, a calcium disodium salt, and a tetrasodium salt, and also include hydrates such dihydrates and tetrahyrates.

With respect to the addition amount of edetic acid or its salt, when it is 0.05 parts by weight or more based on 1 part by weight of famotidine, the stabilization effect is already confirmed. The upper limit is not particularly defined but is a saturated solution concentration of edetic acid or its salt. Preferably, the addition amount of edetic acid or its salt is from 0.05 to 2 parts by weight base on 1 part by weight of famotidine.

In the water-soluble oral solution of the invention, edetic acid or its salt, purified water and an excipient are added to famotidine or its salt, and after dissolution, the pH is adjusted within the range of from 5.5 to 7.0 by an acidic substance or a basic substance.

In the invention, various additives that are used for usual water soluble oral solutions can be contained. Examples include sweeteners, antiseptics, acidulants, flavors, corrigents, thickening agents, and pH regulators. Specific examples of the sweeteners include sugars such as sucrose and glucose syrup; sugar alcohols such as mannitol, sorbitol, and xylitol; synthetic sweeteners such as sodium cyclamate, sodium saccharide, aspartame, sucralose, acesulfame potassium,and stevia; glycyrrhizin and salts thereof; and honey. Specific examples of the antiseptics include 4-hydroxybenzoic acid esters such as methylparaben and propylparaben; and benzoic acids such as sodium benzoate. Specific examples of the acidulants include citric acid, ascorbic acid, and malic acid. Examples of the flavors include menthol, camphor, essential oils such as peppermint oil, mint oil, and cinnamon oil, orange flavor, and drink flavor. Examples of the corrigents include aminoacetic acid and essential oils such as lemon oil and orange oil. Examples of the thickening agents include cellulose derivative such as hydroxypropyl cellulose, hydroxypropylmethyl cellulose, hydroxyethyl cellulose, and carboxymethyl cellulose, polyvinylpyrrolidone, sodium alginate, chondroitin sodium sulfate, powdered agar, gelatin, guar gum, and xanthan gum. Specific examples of the pH regulators include acidic substance such as citric acid, malic acid, lactic acid, tartaric acid, and adipic acid, and salts thereof.

Further, the-oral solution of the invention can contain active ingredients other than famotidine. Examples include antacids, enhancers of gastric mucous membrane control factor, activating agents of digestive tract, vitamins, crude drugs, and stomachics. Specific examples of the antacids include cimetidine, ranitidine, nizatidine, roxatidine acetate, lansoprazole, rabeprazole sodium, omeprazole, scopolia extract, bellandonna extract, isopropamide iodide, scopolamine hydrobromide, oxyphencyclimine hydrochloride, dicyclomine hydrochloride, metixene hydrochloride, methylatropine bromide, scopolamine methylbromide, methyl hyoscyamine bromide, methylbenactyzium, and pirenzepine hydrochloride. Examples of the enhancers of gastric mucous membrane control factor include sucralfate, ecabet sodium, cetraxate hydrochloride, benexate hydrochloride betadex, enprostil, ornoprostil, gefarnate, sofalcone, a thiamine-cobalt-chlorophyll complex compound, teprenone, troxipide, plaunotol, proglumide, polapresinc, irsogladine maleate, misoprostol, clebopride malate, rebamipide, aceglutamide aluminum, urogastrone, azulene derivatives, aldioxa, glycyrrhizinic acid and salts thereof, glycyrrhiza extract, L-glutamine, salts of copper chlorophyllin, histidine hydrochloride, methylmethionine sulfonium chloride, and pig gastric wall disassimilation products; examples of the activating agents of digestive tract include trimebutine maleate, mosapride citrate, cisapride, itopride hydrochloride, metoclopramide, and domperidone; examples of vitamins include vitamins B₁, B₂ and C and derivatives thereof, and their salts, vitamin E, and vitamin D; and examples of the crude drugs include Mallotus japonicus, Corydalis bulbosa, licorice (Glycyrrhiza uralensis Fisch.), aloe, fennel, Isodon japonicus, gentian, cinnamon, Perilla frutescens, Picea jezoensis var. hondoensis, Panax ginseng, common hop, and nux vomica. Examples of stomachics include carnitine chloride, bethanechol chloride, betaine hydrochloride, and glutaminic acid hydrochloride.

### BEST MODE FOR CARRYING OUT THE INVENTION

The invention will be described below in more detail with reference to the following Examples and Test Example, but it should not be construed that the invention is limited thereto.

### Test Example 1:

Purified water (90 mL) was elevated to a temperature of 40°C, in which were then dissolved a stabilizing agent set forth in the following table and 200 mg of famotidine. After cooling, suitable amounts of a 1% citric acid solution and purified water were added to the solution, to prepare 200 mL of a water-soluble oral solution preparation having a pH of 6.5. The residual rates of famotidine when these preparations were preserved at 50°C for 4 weeks are tabulated as follows.

| Stabilizing agent | | Residual rate (%) |
|---|---|---|
| Calcium disodium edetate 100 mg | | 96.5 |
| Aspartic acid | 80 mg | 93.0 |
| | 160 mg | 92.4 |

The water-soluble oral solutions using aspartic acid as the solubilizing agent exhibited a residual rate of famotidine of 92 to 93%, whereas the water-soluble oral solution using an edetic acid salt as the solubilizing agent exhibited a markedly superior residual rate as 96.5%. Incidentally, the reason why the residual rate of the water-soluble oral solution containing an edetic acid salt did not reach slightly 97% resides in the matter that it was preserved under severe conditions at 50°C for 4 weeks.

### Example 1:

Purified water (900 mL) was elevated to a temperature of 40°C, in which were then dissolved 1 g of disodium edetate and 1 g of famotidine, and 2.5 g of mannitol was further added to the solution. After cooling, purified water was added to make 1,000 mL. This water-soluble oral solution preparation had a pH of 6.7 and after preservation at 40°C for 2 months, exhibited a residual rate of famotidine of 97.4%.

### Example 2:

Purified water (900 mL) was elevated to a temperature of 40°C, in which were then dissolved 1 g of disodium edetate and 1 g of famotidine. After cooling, purified water was added to make 1,000 mL. This water-soluble oral solution preparation had a pH of 6.7 and after preservation at 40°C for 2 months, exhibited a residual rate of famotidine of 97.5%.

### Example 3:

Purified water (90 mL) was elevated to a temperature of 40°C, in which were then dissolved 100 mg of calcium disodium edetate and 100 mg of famotidine. After cooling, suitable amounts of a 1% citric acid solution and purified water were added to prepare 100 mL of a water-soluble oral solution having a pH of 6.0. When this preparation was preserved at room temperature for 12 months, it exhibited a residual rate of famotidine of 98.0%.

### Example 4:

Purified water (90 mL) was elevated to a temperature of 40°C, in which were then dissolved 100 mg of calcium disodium edetate and 100 mg of famotidine. After cooling, suitable amounts of a 1% citric acid solution and purified water were added to prepare 100 mL of a water-soluble oral solution having a pH of 6.25. When this preparation was preserved at room temperature for 12 months, it exhibited a residual rate of famotidine of 97.7%.

### Example 5:

Purified water (90 mL) was elevated to a temperature of 40°C, in which were then dissolved 100 mg of calcium disodium edetate and 100 mg of famotidine. After cooling, suitable amounts of a 1% citric acid solution and purified water were added to prepare 100 mL of a water-soluble oral solution having a pH of 6.5. When this preparation was preserved at room temperature for 12 months, it exhibited a residual rate of famotidine of 98.7%.

### Example 6:

Purified water (90 mL) was elevated to a temperature of 40°C, in which were then dissolved 44 mg of methylparaben, 6 mg of propylparaben, 100 mg of calcium disodium edetate, and 100 mg of famotidine. After cooling, suitable amounts of a 1% citric acid solution and purified water were added to prepare 100 mL of a water-soluble oral solution having a pH of 5.75. When this preparation was preserved at room temperature for 12 months, it exhibited a residual rate of famotidine of 98.1%.

### Example 7:

Purified water (90 mL) was elevated to a temperature of 40°C, in which were then dissolved 44 mg of methylparaben, 6 mg of propylparaben, 100 mg of calcium disodium edetate, and 100 mg of famotidine. After cooling, suitable amounts of a 1% citric acid solution and purified water were added to prepare 100 mL of a water-soluble oral solution having a pH of 6.25. When this preparation was preserved at room temperature for 12 months, it exhibited a residual rate of famotidine of 98.0%.

### Example 8:

Purified water (90 mL) was elevated to a temperature of 40°C, in which were then dissolved 44 mg of methylparaben, 6 mg of propylparaben, 100 mg of calcium disodium edetate, and 100 mg of famotidine. After cooling, suitable amounts of a 1% citric acid solution and purified water were added to prepare 100 mL of a water-soluble oral solution having a pH of 7.0. When this preparation was preserved at room temperature for 12 months, it exhibited a residual rate of famotidine of 97.6%.

### Example 9:

Purified water (90 mL) was elevated to a temperature of 40°C, in which were then dissolved 44 mg of methylparaben, 6 mg of propylparaben, 10 mg of calcium disodium edetate, and 100 mg of famotidine, and 15 mg of sucralose was further added to the solution. After cooling, suitable amounts of a 1% lactic acid solution and purified water were added to prepare 100 mL of a water-soluble oral solution having a pH of 6.5. When this preparation was preserved at room temperature for 12 months, it exhibited a residual rate of famotidine of 99.0%.

### Example 10:

Purified water (90 mL) was elevated to a temperature of 40°C, in which were then dissolved 44 mg of methylparaben, 6 mg of propylparaben, 10 mg of calcium disodium edetate, and 50 mg of famotidine, and 15 mg of sucralose was further added to the solution. After cooling, suitable amounts of a 1% lactic acid solution and purified water were added to prepare 100 mL of a water-soluble oral solution having a pH of 6.5. When this preparation was preserved at room temperature for 12 months, it exhibited a residual rate of famotidine of 98.8%.

### INDUSTRIAL APPLICABILITY

The water-soluble oral solution of the invention can be produced by inexpensive and simple steps, and even in a low concentration of famotidine (1 mg/mL or less), it can ensure sufficient stability such that it can endure the market circulation, i.e., sufficient stability such that a residual rate after preservation at room temperature for 12 months is 97% or more.

## Claims

1. A water-soluble oral solution comprising famotidine and edetic acid or a salt thereof.

2. The water-soluble oral solution according to claim 1, which has a pH of from 5.5 to 7.0.

3. The water-soluble oral solution according to claim 1 or 2, which has a concentration of famotidine of from 0.025 to 4 mg/mL.

4. The water-soluble oral solution according to claim 3, which has a concentration of famotidine of from 0.2 to 2 mg/mL.

5. The water-soluble oral solution according to any of claims 1 to 4, wherein edetic acid or its salt is compounded in an amount of from 0.05 to 2 parts by weight based on 1 part by weight of famotidine.
